# EUROPÄISCHE PATENTSCHRIFT

(11) **EP 0 475 300 B1**
(45) Veröffentlichungstag und Bekanntmachung des Hinweises auf die Patenterteilung: **05.07.1995**
(21) Anmeldenummer: 91115110.8
(22) Anmeldetag: 06.09.1991
(51) Int. Cl.: A61C 8/00

(54) **Enossales Implantat für einen festsitzenden Zahnersatz**
Dental implant for an anchored prosthesis
Implant dentaire pour une prothèse dentaire fixe

(30) Priorität: 08.09.1990 DE 4028857
(43) Veröffentlichungstag der Anmeldung: 18.03.1992
(73) Patentinhaber: EBERLE MEDIZINTECHNISCHE ELEMENTE GMBH, D-75449 Wurmberg (DE); IMZ-Fertigungs- und Vertriebsgesellschaft für dentale Technologie mbH, 70794 Filderstadt (DE)
(72) Erfinder: Dürr, Walter, W-7537 Remchingen (DE); Kirsch, Axel, Dr., W-7000 Stuttgart 1 (DE)
(74) Vertreter: Goddar, Heinz J., Dr.

(56) Entgegenhaltungen:
- WO-A-87/06816
- FR-A- 2 580 169

## Beschreibung

Die Erfindung betrifft ein enossales Implantat für einen festsitzenden Zahnersatz, dessen Befestigungseinrichtungen einen Befestigungskopf und einen mit einem Grundkörper über eine Schraubverbindung, ggf. unter Zwischenschaltung eines Distanzteiles, insbesondere aus Metall bestehend, verbindbaren, aus dem offenen distalen Ende des Grundkörpers vorstehenden Implantatpfosten aus Metall aufweisen, wobei der Implantatpfosten nahe dem distalen Rand des Grundkörpers bereichsweise konzentrisch von einem Zwischenelement aus (visko-)elastischem Material, wie Kunststoff, insbesondere Polyoxymethylen, umgeben ist.

Aus der DE-OS 38 39 724 ist ein derartiges enossales Implantat bekannt, bei dem in das distale, offene Ende des Grundkörpers ein mit einer Schulter dem oberen Rand des Grundkörpers entsprechendes Distanzteil aus Metall einschraubbar ist. Das den Implantatpfosten bereichsweise konzentrisch umgebende Zwischenelement aus Kunststoff liegt mit einer Ringschulter an dem dem Zahnersatz zugewandten Rand des Distanzteiles an, wobei der Implantatpfosten unter Andrücken einer dem Zwischenelement zugewandten Anlagefläche des Zahnersatzes an eine dem Distanzteil abgewandte Anlageschulter des Zwischenelementes in das Distanzteil einschraubbar ist. Das gattungsgemäße enossale Implantat weist vorzügliche Dämpfungseigenschaften auf, gewährleistet aber keine befriedigende Verdrehsicherheit des Zahnersatzes gegenüber dem Grundkörper und damit dem Kieferknochen, sofern nicht besondere Maßnahmen, wie Verkleben etc., ergriffen werden. Das bekannte gattungsgemäße Implantat eignet sich daher nur sehr bedingt für die Verwendung als Einzelzahnimplantat; auch bei anderen Anwendungsfällen kann die unbefriedigende Lösung der Verdrehsicherheit stören.

Andererseits ist Gegenstand der DE-OS 39 17 690 (nicht vorveröffentlicht) ein enossales Einzelzahnimplantat für einen festsitzenden Zahnersatz, bei dem das Distanzteil aus einer in den Grundkörper einschraubbaren zweiteiligen Metall-Distanzhülse besteht, deren beide Teile durch Kontern miteinander und mit dem Grundkörper verriegelbar sind, wodurch sich eine verdrehsichere Verbindung zwischen dem Grundkörper und dem Distanzhülsen-Ober- und Unterteil ergibt; sofern für eine entsprechend verdrehsichere Befestigung gesorgt wird, läßt sich demzufolge ein Zahnersatz verdrehsicher an einem der letztgenannten Teile der Distanzhülse verbinden. Das bekannte enossale Einzelzahnimplantat hat sich im Prinzip bewährt, macht jedoch die Verwendung spezieller Konterwerkzeuge erforderlich, die in manchen Anwendungsfällen hinderlich sein kann.

Der Erfindung liegt daher die Aufgabe zugrunde, das gattungsgemäße enossale Implantat dahingehend weiterzubilden, daß mit einfachen Mitteln eine zumindest weitgehende Verdrehsicherheit des Implantatpfostens und damit des Befestigungskopfes desselben gegenüber dem Grundkörper und damit gegenüber dem Kieferknochen bzw. dem Körpergewebe erzielt wird.

Erfindungsgemäß wird diese Aufgabe in Weiterbildung des gattungsgemäßen enossalen Implantates dadurch gelöst, daß das Zwischenelement aus einem Sicherungsring mit im wesentlichen ebenen, glatten Stirnflächen besteht; daß der Grundkörper und/oder das zwischengeschaltete Distanzteil nahe seinem distalen Umfangsrand eine mit einer Anlageschulter für die proximale Stirnfläche des Sicherungsringes versehene Ringausnehmung zum im wesentlichen umfangsmäßig bündigen Aufnehmen zumindest nahe zu der gesamten Längserstreckung des Sicherungsringes aufweist/aufweisen; daß der Implantatpfosten und/oder das Distanzteil eine Ringschulter zur Anlage an die distale Stirnfläche des Sicherungsringes aufweist/aufweisen; und daß zumindest ein Teil der mit dem Sicherungsring beim Einschrauben des Implantatpfostens und/oder des Distanzteiles in den Rundkörper bzw. das Distanzteil in Preßeingriff kommenden Flächen des Grundkörpers und des Implantatpfostens sowie - sofern vorhanden - des Distanzteiles mit Formschlußvertiefungen versehen ist, in die das Material des Sicherungsringes unter dem Einschraubdruck unter Ausbildung einer Formschlußdrehsicherung zwischen den an den Sicherungsring angrenzenden Implantatelementen hineindeformierbar ist.

Dabei kann vorgesehen sein, daß zumindest ein Teil der Formschlußvertiefungen an der Umfangsfläche der Ringausnehmung und/oder des der Ringschultern benachbarten Bereiches des Implantatpfostens und/oder des Distanzteiles vorgesehen ist.

Die Erfindung schlägt auch vor, daß zumindest ein Teil der Formschlußvertiefungen an der Anlageschulter der Ringausnehmung und/oder an der Ringschulter vorgesehen ist.

Ferner kann nach der Erfindung vorgesehen sein, daß die Anlageschulter und/oder die Ringschulter mehrere taschenartige Stirnvertiefungen aufweist.

In weiterer Ausbildung der Erfindung ist vorgesehen, daß vier taschenartige Stirnvertiefungen gleichmäßig in Umfangsrichtung der Anlageschulter und/oder der Ringschulter verteilt sind.

Die Erfindung schlägt auch vor, daß die Anlageschulter und/oder die Ringschulter eine Stirnverzahnung aufweist/aufweisen.

Dabei kann vorgesehen sein, daß die Stirnverzahnung in der Weise sägezahnartig ausgebildet ist, daß sich beim Einschraubvorgang eine das Einschrauben begünstigende und das Herausschrauben benachteiligende Ratschenwirkung bezüglich der anliegenden Stirnfläche des Sicherungsringes ergibt.

Die Erfindung kann ferner dadurch gekennzeichnet sein, daß die Stirnverzahnung kronenartig ausgebildet ist.

Eine weitere Ausführungsform der Erfindung schlägt vor, daß der Implantatpfosten unmittelbar in den Grundkörper einschraubbar ist.

Auch kann nach der Erfindung vorgesehen sein, daß der Implantatpfosten einen polygonalen Befestigungskopf für den Zahnersatz aufweist.

Der Erfindung liegt die überraschende Erkenntnis zugrunde, daß es gelingt, ein weitgehend verdrehsicheres Implantat zu schaffen, in dem ein beim Einschrauben des Implantatpfostens z.B. in den Grundkörper ein aus Kunststoff bestehender Sicherungsring so deformiert wird, daß sich eine formschlüssige Verdrehsicherung im Zusammenwirken mit den Formschlußvertiefungen des z.B. Grundkörpers mit denjenigen des z.B. Implantatpfostens ergibt. Beispielsweise kann dies mittels einer Stirnverzahnung an der proximalen Ringschulter des Implantatpfostens erreicht werden, die zunächst beim Eindrehen des Implantatpfostens noch über den Sicherungsring gleitet, ehe dann später das Material des Sicherungsringes in die Stirnverzahnung unter Deformation eingreift. Entsprechendes wird auch erreicht durch Formschlußvertiefungen an der Anlageschulter der Ringausnehmung des Implantatpfostens oder des Distanzteiles, wobei hier vorzugsweise die Formschlußvertiefungen z.B. durch einzelne Taschen gebildet sein können, die bei bestimmten anderen Anwendungsfällen zum Ansatz eines Werkzeuges oder aber auch zum Herstellen einer Steckverbindung mit weiteren Aufbauelementen dienen.

Der erfindungsgemäße Sicherungsring kann nicht nur zur Erzielung von Verdrehsicherheit zwischen dem Grundkörper und dem unmittelbar in diesen eingeschraubten Implantatpfosten verwendet werden, sondern z.B. auch bei Implantaten, wie sie in der DE-OS 38 39 724 beschrieben sind, d.h. also solchen, bei denen in den Grundkörper zunächst ein Distanzteil und erst in das Distanzteil der Implantatpfosten eingeschraubt ist. Ggf. kann die erfindungsgemäße Verdrehsicherung auch "mehrstufig" vorgesehen sein, also z.B. zwischen Grundkörper und Distanzteil oder -Hülse einerseits und zwischen Distanzteil bzw. -Hülse und dem Implantatpfosten andererseits.

Die Erfindung eignet sich insbesondere auch in Verbindung mit einem verdrehsicheren Aufbau für Einzelzahnimplantate oder dergleichen, wie er Gegenstand der gleichzeitig eingereichten deutschen Patentanmeldung P P 40 28 856.0 ist.

Weitere Merkmale und Vorteile der Erfindung ergeben sich aus den Ansprüchen und aus der nachfolgenden Beschreibung, in der ein Ausführungsbeispiel anhand der schematischen Zeichnungen im einzelnen erläutert ist. Dabei zeigt:
- Fig. 1: ein Ausführungsbeispiel eines enossalen Implantats nach der Erfindung in zusammengebautem Zustand im axialen Längsschnitt;
- Fig. 2: das Implantat von Figur 1 in Explosionsdarstellung;
- Fig. 3: den Implantatpfosten des Implantates von Figur 1 und Figur 2 in der Ansicht von unten; und
- Fig. 4: den Grundkörper des Implantates von Figur 1 und Figur 2 in der Draufsicht.

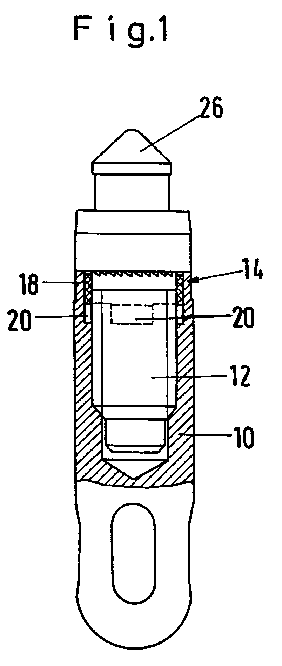

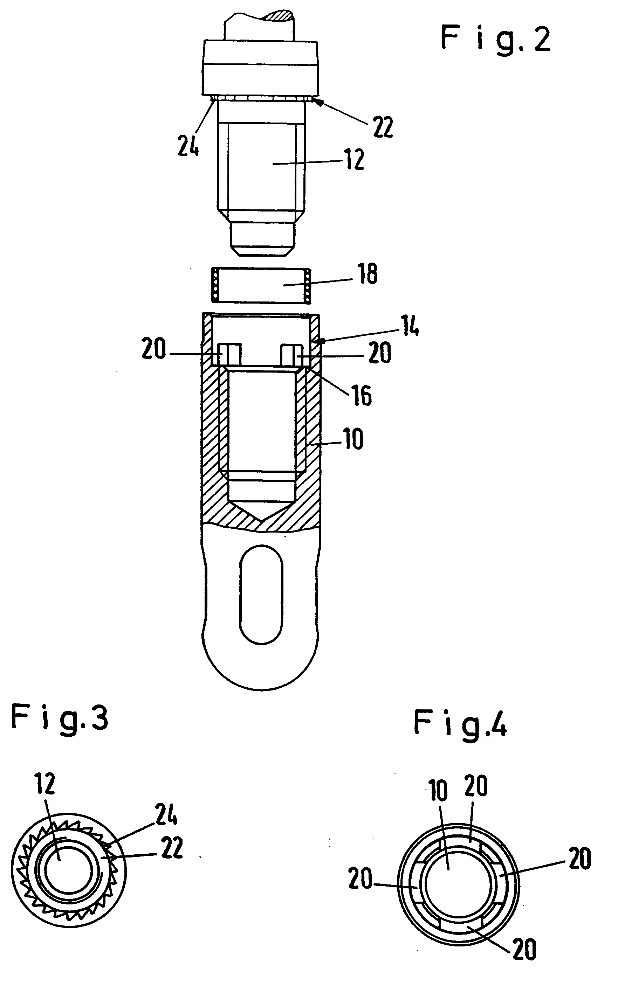

Wie die Zeichnung erkennen läßt, weist das enossale Implantat nach der Erfindung bei dem dort gezeigten Ausführungsbeispiel einen Grundkörper 10 auf, der aus Titan besteht und an seiner mit dem Körpergewebe in Kontakt kommenden Außenfläche mit Hydroxylapatit oder TPS beschichtet ist. In seiner Funktion entspricht dieser Grundkörper demjenigen des Implantats nach der DE-OS 38 39 724, auf die zur weiteren Erläuterung der Wirkungsweise z.B. der Durchbrechungen des Grundkörpers 10 verwiesen wird. In den Grundkörper 10 ist ein Implantatpfosten 12 einschraubbar bzw., in der in Figur 1 gezeigten Endstellung, eingeschraubt. Am distalen, in Figur 1 und 2 oben liegenden Ende des Grundkörpers 10 ist eine Ringausnehmung 14 vorgesehen, die in der Zeichnung nach unten gesehen mit einer Anlageschulter 16 für einen Sicherungsring 18 versehen ist. Der Sicherungsring 18 besteht bei dem gezeigten Ausführungsbeispiel aus Polyoxymethylen. Die Anlageschulter 16 weist vier Stirnvertiefungen 20 auf. Der Implantatpfosten 12 weist eine in der Zeichnung nach unten weisende Ringschulter 22 auf, die mit einer sägezahnartigen, kronenförmig ausgebildeten Stirnverzahnung 24 versehen ist.

Ein Befestigungskopf 26 des Implantatpfostens 12 für einen in der Zeichnung nicht wiedergegebenen Zahnersatz ist bei dem gezeigten Ausführungsbeispiel konisch ausgebildet, kann aber auch z.B. umfangsmäßig polygonale Form haben, wodurch ein verdrehsicheres Aufsetzen eines Zahnersatzes auf den Implantatpfosten 12 möglich ist.

Die Erfindung ist bei dem gezeigten Ausführungsbeispiel dadurch verwirklicht, daß beim Einschrauben des Implantatpfostens 12 in den Grundkörper 10 der aus Kunststoff bestehende Sicherungsring 18 innerhalb der Ringausnehmung 14 immer stärker zusammengedrückt wird. Die Stirnvertiefungen 20 der Anlageschulter 16 der Ringausnehmung 14 einerseits und die Stirnverzahnung 24 der Ringschulter 22 des Implantatpfostens 12 andererseits bilden jeweils Formschlußvertiefungen, in die das Kunststoffmaterial des Sicherungsringes 18 aufgrund seiner viskoelastischen Eigenschaften beim Anziehen des Implantatpfostens 12 immer stärker "hineinfließt", so daß nach Erreichen der Endstellung des Implantatpfostens 12 im Grundkörper 10 eine gegen Verdrehung sichernde Formschlußverbindung zwischen dem Grundkörper 10 und dem Implantatpfosten 12 und damit auch dem Befestigungskopf 26 gewährleistet ist. Dies gibt die Möglichkeit, den Zahnersatz verdrehungssicher im Körpergewebe zu verankern, insbesondere dann, wenn auch der Befestigungskopf 26 noch, wie weiter oben ausgeführt, polygonal, beispielsweise hexagonal, ausgebildet ist. Ungeachtet der auf diese Weise erzielten Verdrehsicherheit läßt sich der Implantatpfosten 12 nichts desto weniger bedarfsweise auch wieder aus dem Grundkörper 10 herausschrauben, wobei der Sicherungsring dann eventuell teilweise zerstört wird, jedoch bei erneutem Eindrehen des Implantatpfostens 12 unter Verwendung eines neuen Sicherungsringes leicht erneut eine Verdrehsicherheit erreicht werden kann.

### Bezugszeichenliste

- 10: Grundkörper
- 12: Implantatpfosten
- 14: Ringausnehmung
- 16: Anlageschulter
- 18: Sicherungsring
- 20: Stirnvertiefung
- 22: Ringschulter
- 24: Stirnverzahnung
- 26: Befestigungskopf

## Patentansprüche

1. Enossales Implantat für einen festsitzenden Zahnersatz, dessen Befestigungseinrichtungen einen Befestigungskopf (26) und einen mit einem Grundkörper (10) über eine Schraubverbindung, ggf. unter Zwischenschaltung eines Distanzteiles, inbesondere aus Metall bestehend, verbindbaren, aus dem offenen distalen Ende des Grundkörpers (10) vorstehenden ImPlantatpfosten (12) aus Metall aufweisen, wobei der Implantatpfosten nahe dem distalen Rand des Grundkörpers bereichsweise konzentrisch von einem Zwischenelement aus (visko-)elastischem Material, wie Kunststoff, insbesondere Polyoxymethylen, umgeben ist, dadurch gekennzeichnet, daß das Zwischenelement aus einem Sicherungsring (18) mit im wesentlichen ebenen, glatten Stirnflächen besteht; daß der Grundkörper (10) und/oder das zwischengeschaltete Distanzteil nahe seinem distalen Umfangsrand eine mit einer Anlageschulter (16) für die proximale Stirnfläche des zugeordneten Sicherungsringes versehene Ringausnehmung (14) zum im wesentlichen umfangsmäßig bündigen Aufnehmen zumindest nahe zu der gesamten Längserstreckung des Sicherungsringes (18) aufweist/aufweisen; daß der Implantatpfosten (12) und/oder das Distanzteil eine Ringschulter (22) zur Anlage an die distale Stirnfläche des Sicherungsringes (18) aufweist/aufweisen; und daß zumindest ein Teil der mit dem Sicherungsring (18) beim Einschrauben des Implantatpfostens (12) und/oder des Distanzteiles in den Grundkörper (10) bzw. das Distanzteil in Preßeingriff kommenden Flächen des Grundkörpers (10) und des Implantatpfostens (12) sowie - sofern vorhanden - des Distanzteiles mit Formschlußvertiefungen (20, 24) versehen ist, in die das Material des Sicherungsringes (18) unter dem Einschraubdruck unter Ausbildung einer Formschlußdrehsicherung zwischen den an den Sicherungsring (18) angrenzenden Implantatelementen (10, 12) hineindeformierbar ist.

2. Implantat nach Anspruch 1, dadurch gekennzeichnet, daß zumindest ein Teil der Formschlußvertiefungen (20, 24) an der Umfangsfläche der Ringausnehmung (14) und/oder des der Ringschulter (22) benachbarten Bereiches des Implantatpfostens (12) und/oder des Distanzteiles vorgesehen ist.

3. Implantat nach Anspruch 1 oder 2, dadurch gekennzeichnet, daß zumindest ein Teil der Formschlußvertiefungen (20, 24) an der Anlageschulter (16) der Ringausnehmung (14) und/oder an der Ringschulter (22) vorgesehen ist.

4. Implantat nach Anspruch 3, dadurch gekennzeichnet, daß die Anlageschulter (16) und/oder die Ringschulter (22) mehrere taschenartige Stirnvertiefungen (20) aufweist.

5. Implantat nach Anspruch 4, dadurch gekennzeichnet, daß vier taschenartige Stirnvertiefungen (20) gleichmäßig in Umfangsrichtung der Anlageschulter (16) und/oder der Ringschulter (22) verteilt sind.

6. Implantat nach einem der Ansprüche 3 bis 5, dadurch gekennzeichnet, daß die Anlageschulter (16) und/oder die Ringschulter (22) eine Stirnverzahnung (24) aufweist/aufweisen.

7. Implantat nach Anspruch 6, dadurch gekennzeichnet, daß die Stirnverzahnung (24) in der Weise sägezahnartig ausgebildet ist, daß sich beim Einschraubvorgang eine das Einschrauben begünstigende und das Herausschrauben benachteiligende Ratschenwirkung bezüglich der anliegenden Stirnfläche des Sicherungsringes (18) ergibt.

8. Implantat nach Anspruch 6 oder 7, dadurch gekennzeichnet, daß die Stirnverzahnung (24) kronenartig ausgebildet ist.

9. Implantat nach einem der vorangehenden Ansprüche, dadurch gekennzeichnet, daß der Implantatpfosten (12) unmittelbar in den Grundkörper (10) einschraubbar ist.

10. Implantat nach einem der vorangehenden Ansprüche, dadurch gekennzeichnet, daß der Implantatpfosten (12) einen polygonalen Befestigungskopf (26) für den Zahnersatz aufweist.

## Claims

1. An endo-osseous implant for a permanently fixed dental prosthesis with securing means comprising a head (26) and a metal implant post (12) projecting from the open distal end of a base member (10) and connectable to the base member (10) by a screw connection, optionally with interposition of a spacer part, more particularly of metal, parts of the post near the distal edge of the base member being concentrically surrounded by an intermediate element made of (visco-)elastic material such as plastics, more particularly polyoxymethylene, characterised in that the intermediate element comprises a securing ring (18) with substantially smooth flat end faces; the base member (10) and/or the interposed spacer part, near its distal peripheral edge, has an annular recess (14) formed with a shoulder (16) for abutting the proximal end-face of the associated securing ring and for the purpose of substantially peripherally flush reception of the securing ring over at least nearly all its total longitudinal extent; the implant post (12) and/or the spacer part has an annular shoulder (22) for abutting the distal end-face of the securing ring (18); and at least a part of the surfaces of the base member (10), the post (12), and the spacer part, if present, coming into pressure engagement with the securing ring (18) when the post (12) and/or the spacer part is screwed into the base member (10) or the spacer part are formed with positive recesses (20, 24) into which the material of the securing ring (18) can be deformed under the screwing-in pressure, resulting in a positive means for preventing twisting between the implant components (10, 12) adjoining the securing ring (18).

2. An implant according to claim 1, characterized in that at least some of the positive recesses (20, 24) are provided on the peripheral surface of the annular recess (14) and/or of the region of the post (12) adjacent the annular shoulders (22) and/or of the spacer part.

3. An implant according to claim 1 or 2, characterised in that at least some of the positive recesses (20, 24) are provided on the abutment shoulder (16) of the annular recess (14) and/or on the annular shoulder (22).

4. An implant according to claim 3, characterized in that the abutment shoulder (16) and/or the annular shoulder (22) have a number of pocket-like recesses (20) in their end-faces.

5. An implant according to claim 4, characterized in that four pocket-like end-face recesses (20) are uniformly distributed in the peripheral direction of the abutment shoulder (16) and/or of the annular shoulder (22).

6. An implant according to any of claims 3 to 5, characterized in that the abutment shoulder (16) and/or the annular shoulder (22) have end-face teeth (24).

7. An implant according to claim 6, characterised in that the end-face teeth (24) are sawtooth-like, so as to produce a ratchet effect relatively to the adjacent end face of the securing ring (18) during the screwing-in process, thus facilitating screwing-in and making unscrewing difficult.

8. An implant according to claim 6 or 7, characterised in that the end-face teeth (24) are contrate.

9. An implant according to any of the preceding claims, characterised in that the implant post (12) is directly screwable into the base member (10).

10. An implant according to any of the preceding claims, characterised in that the post (12) has a polygonal head (26) for securing the dental prosthesis.

## Revendications

1. Implant intra-osseux pour une prothèse dentaire fixe, dont les dispositifs de fixation comprennent une tête de fixation (26) et une tige d'implant (13) en métal qui peut être reliée à un corps de base (10) par une liaison vissée, le cas échéant en intercalant une pièce d'écartement, réalisée en particulier en métal, et qui dépasse de l'extrémité distale ouverte du corps de base (10), la tige d'implant étant entourée, à proximité du bord distal du corps de base, sous forme de secteurs concentriques, d'un élément intermédiaire en matériau (visco-)élastique, tel qu'en matière plastique, en particulier en polyoxyméthylène, caractérisé en ce que l'élément intermédiaire est constitué d'un anneau de blocage (18) dont les surfaces frontales sont sensiblement planes et lisses ; en ce que le corps de base (10) et/ou la pièce d'écartement intercalée comporte, à proximité de son bord périphérique distal, un évidement annulaire (14) qui est pourvu d'un épaulement d'appui (16) pour la surface frontale proximale de l'anneau de blocage et qui sert à recevoir, de manière sensiblement jointive en périphérie, l'anneau de blocage (18) sur au moins la quasi-totalité de son extension longitudinale ; en ce que la tige d'implant (12) et/ou la pièce d'écartement comporte un épaulement annulaire (22) destiné à venir en appui contre la surface frontale distale de l'anneau de blocage (18) ; et en ce qu'au moins une partie des surfaces qui appartiennent au corps de base (10) et à la tige d'implant (12) ainsi qu'à la pièce d'écartement - éventuelle - et qui coopèrent à la pression avec l'anneau de blocage (18) lors du vissage de la tige d'implant (12) et/ou de la pièce d'écartement dans le corps rond (10) ou dans la pièce d'écartement est pourvue de renfoncements de liaison par complémentarité de formes (20, 24), dans lesquels le matériau de l'anneau de blocage (18) peut pénétrer sous la pression de vissage en formant un blocage en rotation par complémentarité de formes entre les éléments d'implants (10, 12) adjacents à l'anneau de blocage (18).

2. Implant selon la revendication 1, caractérisé en ce qu'au moins une partie des renfoncements de liaison par complémentarité de formes (20, 24) se trouve sur la surface périphérique de l'évidement annulaire (14) et/ou de la zone appartenant à la tige d'implant (12) et/ou à la pièce d'écartement et voisine de l'épaulement annulaire (22).

3. Implant selon la revendication 1 ou 2, caractérisé en ce qu'au moins une partie des renfoncements de liaison par complémentarité de formes (20, 24) se trouve sur l'épaulement d'appui (16) de l'évidement annulaire (14) et/ou sur l'épaulement annulaire (22).

4. Implant selon la revendication 3, caractérisé en ce que l'épaulement d'appui (16) et/ou l'épaulement annulaire (22) comporte plusieurs renfoncements frontaux (20) en forme de poches.

5. Implant selon la revendication 4, caractérisé en ce que quatre renfoncements frontaux (20) en forme de poches sont répartis à intervalles réguliers dans le sens circonférentiel de l'épaulement d'appui (16) et/ou de l'épaulement annulaire (22).

6. Implant selon l'une des revendications 3 à 5, caractérisé en ce que l'épaulement d'appui (16) et/ou l'épaulement annulaire (22) comporte une denture frontale (24).

7. Implant selon la revendication 6, caractérisé en ce que la denture frontale (24) est conçue en dents de scie, de façon à obtenir, lors du vissage, un effet de crantage favorable au vissage et défavorable au dévissage par rapport à la surface frontale adjacente de l'anneau de blocage (18).

8. Implant selon la revendication 6 ou 7, caractérisé en ce que la denture frontale (24) est conçue en forme de couronne.

9. Implant selon l'une des revendications précédentes, caractérisé en ce que la tige d'implant (12) est vissée directement dans le corps de base (10).

10. Implant selon l'une des revendications précédentes, caractérisé en ce que la tige d'implant (12) comporte une tête de fixation polygonale (26) pour la prothèse dentaire.
